# EUROPEAN PATENT SPECIFICATION

(11) **EP 0 565 560 B1**
(45) Date of publication and mention of the grant of the patent: **28.09.1994**
(21) Application number: 92901671.5
(22) Date of filing: 03.01.1992
(51) Int. Cl.: A61K 9/28, A61K 9/20

(54) **METHOD FOR PRODUCTION OF SOLID PHARMACEUTICAL PREPARATION**
VERFAHREN ZUR HERSTELLUNG EINER FESTEM PHARMAZEUTISCHEN ZUSAMMENSETZUNG
PROCEDE DE PRODUCTION D'UNE PREPARATION PHARMACEUTIQUE SOLIDE

(30) Priority: 03.01.1991 GB 9100040; 14.08.1991 GB 9117525
(43) Date of publication of application: 20.10.1993
(73) Proprietor: GLAXO CANADA INC., Mississauga, Ontario L5N 6L4 (CA)
(72) Inventor: MOLLOY, Thomas, Patrick, Kitchener, Ontario N2N 1Z6 (CA); LEE, David, Thornhill, Ontario L3T 5W3 (CA); CHOPRA, Sham, Kumar, Brampton, Ontario L6T 2L5 (CA)
(74) Representative: Brewer, Christopher Laurence, Dr.
(86) International application number: CA9200004
(87) International publication number: WO9211845

(56) References cited:
- EP-A- 0 035 780
- EP-A- 0 063 014
- EP-A- 0 135 829
- EP-A- 0 153 105
- EP-A- 0 365 123
- WO-A-85/03636
- GB-A- 2 170 104
- US-A- 2 841 528
- US-A- 2 852 433
- US-A- 3 325 365

## Description

The present invention relates to a method of preparing film coated solid pharmaceutical preparations, for example, pills or tablets which avoids the need to use water or organic solvents in the coating process, and to pharmaceutical preparations prepared by said process. More particularly the invention relates to a method of preparing pharmaceutical preparations with enteric, macroporous or microporous coatings or coated with semipermeable membranes.

Many industrial coating processes involve the use of organic solvents. Such solvents, for example acetone, methanol, dichloromethane and ethyl acetate, are relatively expensive, potentially dangerous and give rise to problems of safe disposal. Some of these solvents are very flammable and must be handled carefully in explosion - proof rooms. Others are considered toxic and therefore workers' exposure to them and their release into the environment must be restricted. In the case of pharmaceutical preparations, there is also concern about the toxicity potential of residual traces of organic solvents in the coating.

Aqueous based coating dispersions eliminate many of the disadvantages associated with organic solvent based coating. The presence of water, however, causes stability problems for compounds that are sensitive to moisture. Another disadvantage for preparations containing very water soluble compounds is that the rate of application of the coating dispersion must be slow and the water evaporation rate high, necessitating relatively high drying temperatures and a slow coating process.

Another disadvantage of conventional coating procedures is the difficulty of consistently obtaining a uniform coat.

Using conventional coating techniques, the porosity of the resulting coat is to a considerable extent related to the rate of evaporation of the solvent used. The rate of evaporation must be closely controlled if coatings are to be prepared free from pores or cracks. This is particularly important where enteric coatings are concerned where the presence of pores or cracks would permit penetration of gastric fluids resulting in premature release of the drug.

The thickness of film coatings generally achievable by conventional techniques, e.g. spray coating is limited which in turn limits the control over the release profile one may achieve with film coated controlled release preparations such as enterically coated preparations.

Semipermeable membranes are used in osmotically controlled drug delivery systems. These are essentially tablets comprising active ingredients and, if required, one or more osmotic agents, encased within the semipermeable membrane which is pierced with an orifice of appropriate size to allow delivery of the drug.

Macroporous and microporous membranes are used in diffusion controlled drug delivery systems which are of two types, reservoir and matrix. Reservoir systems comprise a water-soluble drug core surrounded by a macroporous or microporous polymer membrane whereas, in matrix systems, the drug is distributed uniformly throughout an inert polymeric matrix.

The microporous membrane is formed with a polymer permeable to drug and the macroporous membrane is formed with a combination of a polymer impermeable to drug and a pore former which dissolves out of the membrane in the presence of water giving rise to macropores or small channels allowing drug transport.

Using conventional coating techniques such as spray-coating, there is a limit to the thickness of semipermeable and macroporous membranes that can be obtained whilst still retaining the coatings' semipermeable properties or ensuring that the macropores do not become blocked. Such semipermeable and macroporous membranes are therefore necessarily thin which limits the strength of the coating and renders the dosage forms vulnerable to splitting, resulting in dose dumping.

Compression coating techniques are known in the art and such techniques overcome the hazardous aspects of using organic solvents and the stability problems of aqueous coating procedures, however, the coats formed by such processes are brittle, rigid and extremely porous. Film coated pharmaceutical preparations have hitherto not been produced by compression coating.

There is, therefore, a need to provide a film coating technique which eliminates the hazardous aspects of using organic solvents, dispenses with the stability problems of aqueous coating procedures and avoids problems of nonuniformity of coat.

There is also a need to provide a convenient film coating technique which provides continuous films free from pores and cracks and which enables film coats to be produced with reasonable thickness.

Accordingly, the present invention provides a method (A) for the production of a solid pharmaceutical preparation where a solid core is coated with a film coat which comprises the following steps:
1a. mixing or granulating finely divided particles of a membrane-forming amorphous or substantially amorphous polymer with a plasticizer which dissolves the polymer;
2a. encasing said solid core with the polymer mix;
3a. heating the polymer-encased material to a temperature at which the amorphous or substantially amorphous polymer forms a continuous film.

This method is suitable for preparing tablets for immediate, delayed or controlled release of the active ingredient. The thickness of the film is varied as required. For immediate release (i.e. within 30 minutes of administration) of drug from the tablet, the thickness of the film coat will generally be 1mm or less, e.g. 0.5 to 1 mm. For delayed release (i.e. release 30 minutes or more after administration), the coat thickness will generally be more than 1mm, e.g. 1.1 to 5mm.

When applied to the preparation of controlled release drug delivery systems method (A) is particularly suitable for preparing systems of both the osmosis and reservoir diffusion type, and also enterically coated pharmaceutical preparations.

The drilling of orifices in osmotic systems is conventionally carried out as a separate step, subsequent to coating, using laser beams or high speed mechanical drills. Use of mechanical drills is impractical for large scale manufacture. Holes formed using laser beams are not always uniform and centered. Besides being extremely expensive, laser drilling equipment can misfire, resulting in undrilled tablets.

According to the process of the present invention osmotic or diffusion cores can be uniformly film coated and, concomitant with the coating process, orifices can be made in the coating without the use of expensive drilling equipment.

Thus in a preferred aspect method (A) may be used to coat osmotic or diffusion cores with semipermeable or microporous membranes whereby the polymer coating is optionally pierced at one or more points.

It will be appreciated that where a core is encased within a semipermeable membrane the piercing of the coating of polymer mix is obligatory and where a core is encased within a microporous membrane the piercing of the coating of polymer mix is optional.

"Diffusion core" when discussed hereinbefore and hereinafter relates to a non-osmotically active core containing a water-soluble drug, or a water-insoluble drug together with a suitable solubilising agent, optionally in combination with conventional pharmaceutical excipients which will themselves preferably be water-soluble.

"Osmotic core" when discussed hereinbefore and hereinafter relates to an osmotically active core which will contain an osmotically active drug, or an osmotically inactive drug in combination with an osmotically active salt (e.g. sodium chloride) optionally in combination with conventional pharmaceutical excipients.

In a further or alternative aspect the invention provides a method (B) for the production of a solid pharmaceutical preparation which comprises the following steps:
1b. mixing together finely divided particles or granules of a medicament, or granules containing a medicament and excipients, and appropriate amounts of a finely divided amorphous or substantially amorphous coating polymer and a plasticizer which dissolves the polymer;
2b. compressing the mixture into tablets;
3b. heating the tablets to a temperature at which fusion of the polymer particles occurs.

Optionally the tablets before or after heating may be film coated according to method (A) described above for coating solid pharmaceutical preparations. If an exterior coating is applied in this way, step 3b. may optionally be omitted.

This method allows particles or granules of the active ingredient to be film coated within the core of the tablet, thereby allowing controlled release of the active component. The onset of the controlled release of active ingredient can be delayed by subjecting the tablet to a further coating step.

Method (B) is particularly suitable for preparing diffusion controlled drug delivery systems of the matrix type (in which case the medicament will preferably be water-soluble) and controlled enteric release formulations.

Suitable amorphous or substantially amorphous polymers for use in the methods of the present invention are those which can be supplied in powdered form and which are capable of forming films. The nature of the polymer employed will depend upon the nature of the coating required. For example, it may be desirable to provide an enteric, a semi-permeable, microporous or macroporous coating. For certain applications it may be desirable to use a polymer soluble in both water and organic solvents or a polymer soluble in organic solvent but not in water.

Polymers that can produce satisfactory enteric films include cellulose acetate phthalate, Aquateric R (a water dispersible formulation of cellulose acetate phthalate), polyvinyl acetate phthalate, hydroxypropyl methylcellulose phthalate 50 (Type NF), hydroxypropyl methylcellulose phthalate 55 (Type NF), and copolymers of methacrylic acid and methacrylic acid methyl ester.

Examples of polymers soluble in both water and organic solvents include pyrrolidone/vinyl acetate copolymer, polyethylene glycol, hydroxypropylcellulose and vinylpyrrolidone/vinyl acetate copolymer.

Examples of polymers soluble in organic solvents but not in water include methylvinylpyridine/methyl acrylate methacrylate copolymers, cellulose acetate, cellulose triacetate, cellulose acetate butyrate, acetylcellulose, nitrocellulose, polyvinyl acetate and shellac.

Polymers which can produce semi-permeable, microporous or macroporous films include, for example, cellulose acetate, cellulose diacetate, cellulose triacetate and cellulose acetate butyrate, ethylcellulose, ethylcellulose acetate, polyethylene glycol, hydroxypropyl cellulose, hydroxypropyl methylcellulose, methylcellulose poly (DL-lactide-co-glycolide), poly (DL-lactide), cellulose acetate phthalate and polyvinyl pyrrolidone.

In a preferred aspect, the amorphous or substantially amorphous polymer will be biodegradable and suitable biodegradable polymers include polyglycolide, poly (DL-lactide-co-glycolide), poly (L-lactide), poly(DL-lactide), caprolactone, (polyanhydrides, poly(orthoesters) and poly(amino acids).

A single one of these polymers or a mixture of two or more may be used.

One group of particularly preferred polymers for use in the methods of the present invention include cellulose acetate phthalate, hydroxypropyl methylcellulose phthalate 55, polyvinyl acetate phthalate, Aquateric, hydroxypropyl methylcellulose phthalate 50, and copolymers of methacrylic acid and methacrylic acid methyl ester.

Preferred polymers for producing semi-permeable, microporous or macroporous coatings for use in the methods of the invention are cellulose acetate (e.g. containing 32.0% or 39.8% acetyl groups), hydroxypropyl cellulose and polyethylene glycol (400 or 4000).

Preferred combinations of said polymers are cellulose acetate (39.8%)/hydroxypropyl cellulose/polyethylene glycol 400; cellulose acetate (39.8%)/cellulose acetate (32.0%)/polyethylene glycol 4000; and cellulose acetate/polyethylene glycol 4000.

A particularly preferred polymer for producing semi-permeable, microporous or macroporous coatings is cellulose acetate, for example cellulose acetate containing 39.8% acetyl groups.

Plasticizers suitable for use in the methods of the invention include water, Diacetin (glyceryl diacetate), Triacetin (glyceryl triacetate), diethyl phthalate, dibutyl phthalate, glycerol tributyrate, triethyl citrate, acetyl triethyl citrate, ethyl lactate, polyethylene glycols, propylene glycol, propylene carbonate, diethyl tartrate, ethylene glycol monoacetate and dibutyl sebacate.

A single one of the plasticizers or a mixture of two or more may be used.

If a combination of water and a water immiscible plasticizer is used, their emulsion is formed with the help of a surfactant before mixing with the polymer(s). Either all, none, or a portion of the water, is evaporated from the polymer mixture prior to encasing the cores.

Plasticizers for use in the methods of the invention may be solids or liquids, but individual plasticizers or combinations of plasticizers which are sparingly soluble and are solid under ambient conditions are preferred.

Plasticizers used in the methods of the invention must be capable of dissolving the chosen polymer. A person skilled in the art would have no difficulty in selecting suitable combinations of polymers and plasticizers for use in the present invention. Conveniently, the combination of polymer and plasticizer will be chosen such that solubilization takes place at a temperature below 100°C.

One group of preferred polymer/plasticizer combinations for use in the methods of the invention include : cellulose acetate phthalate/glyceryl triacetate; hydroxypropyl methylcellulose-phthalate/diethyl phthalate; copolymers of methacrylic acid and methacrylic acid methyl ester/propylene glycol; hydroxypropyl methylcellulose phthalate/glyceryl triacetate; hydroxypropyl methylcellulose phthalate/ethyl lactate; hydroxypropyl methylcellulose phthalate/triethyl citrate; cellulose acetate phthalate/diethyl phthalate, Aquateric/diethyl phthalate, Aquateric/glyceryl triactate, polyvinyl acetate phthalate/diethyl phthalate and hydroxypropyl methylcellulose phthalate/dibutyl sebacate.

Preferred polymer/plasticizer combinations for preparing semi-permeable, microporous or macroporous coatings include cellulose acetate/glyceryl triacetate.

Particularly preferred combinations of polymers and plasticizers for the formation of enteric coatings according to the method of the invention include : cellulose acetate phthalate/glyceryl triacetate; hydroxypropyl methylcellulose phthalate/diethyl phthalate; copolymers of methacrylic acid and methacrylic acid methyl ester/propylene glycol; hydroxypropyl methylcellulose phthalate/glyceryl triacetate; hydroxypropyl methylcellulose phthalate/ethyl lactate; hydroxypropyl methylcellulose phthalate/triethyl citrate; cellulose acetate phthalate/diethyl phthalate, Aquateric/diethyl phthalate, Aquateric/glyceryl triacetate, polyvinylacetate phthalate/diethyl phthalate and hydroxypropyl methylcellulose phthalate/dibutyl sebacate.

Water insoluble polymers may be mixed with the enteric polymer if a relatively long delay in drug release is required. Water insoluble polymers which may be mixed with enteric polymer include ethyl cellulose, cellulose acetate, cellulose triacetate, cellulose acetate butyrate, polyethylene and polypropylene.

Preferably the plasticizer will be such as to lower the glass transition temperature and melting temperature of the chosen polymer.

The amount of plasticizer used will vary depending on the polymer or polymers chosen.

Conveniently the weight of plasticizer used will be less than one half of the weight of the polymer (e.g. one third).

Optionally one or more surfactants may be mixed or granulated together with the polymer and plasticizer. Any of the anionic, cationic or non-ionic surfactants conventionally used in pharmacy may be employed. Such surfactants include, for example, sulphated monoglyceride, benzalkonium chloride and polyoxyethylene sorbitol acid esters (e.g. polyoxyethylene sorbitan mono-oleate).

A particularly preferred surfactant for use in the methods of the present invention is polyoxyethylene sorbitan mono-oleate.

Lubricants may be used to improve the flow properties of the granulated polymers. Suitable lubricants for use in the methods of the invention include magnesium stearate, stearic acid, sterotex, carbowax 6000, polyethylene glycol 4000, sodium lauryl sulphate and Myvatex TL.

Colouring agents, perfumes, pigments, waxes and other conventional additives may be incorporated into the coating or into any ingredient of the coating if desired.

Where pore formers are required, for example in the production of macroporous polymer coatings, these may he incorporated into the polymer mix at any convenient stage prior to the encasing step. Examples of suitable pore formers include sodium chloride, sorbitol, mannitol and low and high molecular weight polyethylene glycols.

Once all the desired ingredients of the polymer mix (e.g. the polymer and plasticizer) have been combined, if the mixture is "wet" and non-flowable the mixture is allowed to stand at room temperature before being used to encase the solid pharmaceutical preparations in order to increase the flowability of the system. The 'standing' time necessary will depend on the particular composition of the polymer mix but will generally be 24 hours minimum.

The temperature to which the polymer encased solid pharmaceutical formulation must be heated in order to obtain a continuous film will vary according to the polymer or polymers, pore former or pore formers and plasticizer or plasticizers selected. Suitable temperatures will be apparent to one skilled in the art. Preferably the temperature will be in the range 60 to 200°C, most preferably about 80°C to 140°C, e.g. 100-120°C.

Typically a heating period of from about 1 to about 30 (such as 5 to 20, e.g. 10) minutes will be sufficient to effect fusion of the polymer particles.

The osmotic and diffusion cores used in the process according to the invention may be prepared according to conventional procedures.

Medicaments which may be suitably coated according to the present invention so as to provide controlled release formulations include those classes of medicaments which are conventionally delivered in controlled release form and those classes of medicaments for which controlled release would be advantageous. Examples of such medicaments include anti-ulcer agents, anti-asthmatic agents (e.g. salbutamol), anti-inflammatory agents, anti-Parkinsonism agents, analgesic agents, diuretic agents, anti-emetics and anti-psychotics.

Medicaments which may advantageously be enterically coated according to the invention include compounds which are irritant to the stomach (e.g. aspirin, bisacodyl) and acid labile compounds (e.g. erythromycin, p-aminosalicylic acid).

Encasing the solid pharmaceutical preparation with the granulated polymer mix may be achieved using any conventional means, preferably using a compression coating machine.

Either during or subsequent to the encasing of the osmotic or diffusion cores with the polymer mix, one or more orifices are optionally made in the amorphous or substantially amorphous polymer casing by piercing using, for example, a pin, or pins, of appropriate diameter.

Preferably the encasing of the osmotic or diffusion cores and the piercing of the orifices in the casing will be carried out in a single operation. This may be achieved, for example, using a compression coating machine with specially designed toolings.

Most preferably the encasing and orifice-making operations according to the present invention will be carried out simultaneously using a compression coating machine wherein either the upper or the lower punch is provided with a pin, or pins, of appropriate length and diameter to make a hole, or holes, of the desired dimensions in the amorphous or substantially amorphous polymer coating.

The pin, or pins, may be machined into the tip face of the punch, in which case they will be of predetermined length.

Alternatively a bore, or bores, may be drilled through the punch suitable to accommodate a shaft, or shafts, having at one end a pin. In use, the pin(s) will protrude beyond the tip face by an amount which can be varied by movement of the shaft(s) within the bore(s). Means are provided by securing the shaft(s) at a desired position within the bore(s).

In either case, the length of the pin(s) protruding beyond the tip surface of the punch will preferably be in the range of 0.1mm to 2.0mm (e.g. 0.1mm to 1.0mm).

Conveniently the pin(s) will be cylindrical. Preferably the pin(s) will be tapered towards the free end.

Preferably the coating of polymer mix will be pierced at one point only, for example using a single pin.

The punch faces may be of any suitable shape and may have a flat or bevelled edge.

The punches will now be described in more detail with reference to the accompanying drawings wherein:
Figure 1 is a cross-sectional view of an upper punch for a single stroke machine having a fixed pin.
Figure 2 is a cross-sectional view of an upper punch for a single stroke machine having an adjustable pin.
Figure 3 is a front elevation of an upper punch for a rotary machine having a fixed pin.

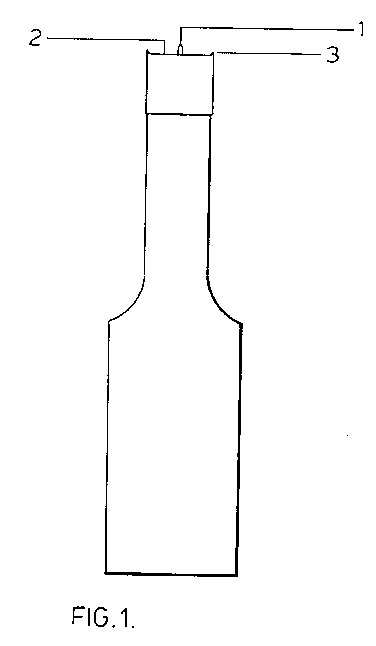

With reference to Figure 1, a pin (1) is located centrally of the tip face (2). The tip face (2) has a bevelled edge (3).

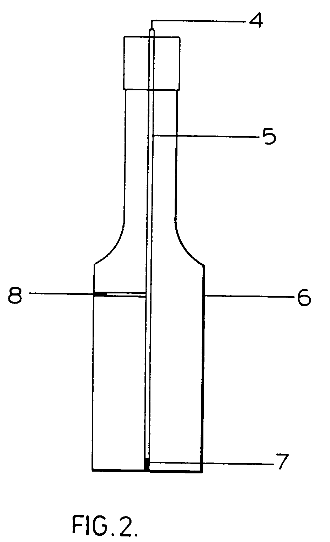

With reference to Figure 2, a pin (4) is mounted terminally of a shaft (5) which shaft (5) is accommodated in a bore which passes through the body of the punch (6). An adjusting screw (7) is provided at the end of the shaft (5) distant from the pin (4). A retaining screw (8) is located in a second bore which is perpendicular to the shaft (5).

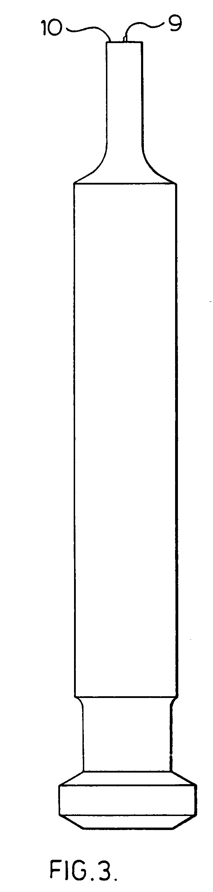

With reference to Figure 3, a pin (9) is located centrally of the tip face (10).

There is thus provided, as a further or alternative aspect of the present invention, a punch for a compression coating machine characterised in that the tip face is provided with a pin.

It is an advantage of the present invention that orifices are formed in the membrane coating of the osmotically controlled drug delivery systems reliably and with reproducible dimensions.

Thus in a further or alternative aspect the present invention provides a plurality of osmotically controlled drug delivery systems wherein each osmotically controlled drug delivery system contains an orifice, or orifices, of substantially identical dimensions.

It will be appreciated that the present invention is suitable for preparing drug delivery systems comprising an osmotic core encased within a microporous polymer membrane which membrane optionally contains an orifice, or orifices.

In such a drug delivery system drug release is controlled by a combination of diffusion and osmosis.

It is frequently desirable that solid pharmaceutical preparations should have an exact thickness of coat. Thus, for example, it may be necessary to control the time of drug release. In order that the time of drug release from a coated solid pharmaceutical preparation be controlled it is necessary that each preparation is accurately coated to a pre-determined thickness; that is to say:
i) the coating thickness on each individual preparation should be uniform; and
ii) the weight of polymer coat on each preparation should be effectively constant as between individual preparations.

The method of the invention allows solid pharmaceutical preparations to be film coated with an accurately known weight of polymer.

Thus in a further or alternative aspect the present invention provides a plurality of film coated solid pharmaceutical preparations wherein the maximum variation in weight of the polymer coat does not exceed ; 5% of the mean weight of polymer coat.

A plurality of film coated solid pharmaceutical preparations means a production run of such preparations, or a course prescribed by a medical practitioner, or a bottle, container, packet or batch of such preparations.

The methods of the invention further allow the polymer coating of each individual solid pharmaceutical preparation to be evenly distributed over the surface of that preparation.

Thus the invention further provides a film coated solid pharmaceutical preparation wherein the difference in thickness between the thinnest part of the coat and the thickest part of the coat is no more than 5% of the mean coating thickness.

It is an advantage of the present invention that solid pharmaceutical preparations can be film coated to a thickness of from about 0.1mm (e.g 0.5mm) to about 5mm or more without any increase in the processing time. This is in contrast to conventional coating processes where not only does the processing time increase with increasing film thickness, but also the risks of solvent entrapment, film cracking and peeling increase.

It is a further advantage of the invention that solid pharmaceutical preparations can be film coated with semipermeable and macroporous membranes to a thickness of from about 0.5mm to about 1mm whilst retaining the functionality of the coats.

A further advantage of the invention is that enterically film coated solid pharmaceutical preparations are provided where the film coat is of a thickness (e.g. 1 to 2mm) such that increased resistance to intestinal fluid is achieved (e.g. 15 minutes to 7 hours).

In a particularly preferred aspect, the present invention provides an enterically film coated solid preparation containing an acid-labile medicament wherein the maximum difference between the thinnest part of the coat and the thickest part of the coat is no more than 5% of the mean coating thickness.

In a further particularly preferred aspect, the invention provides a plurality of enterically coated solid pharmaceutical preparations containing an acid-labile medicament wherein the maximum variation in weight of the polymer coat does not exceed ; 5% of the mean weight of polymer coat.

The invention is further illustrated by the following non-limiting examples.

### Example 1

A water dispersible formulation of cellulose acetate phthalate (Aquateric) (85g) and glyceryl triacetate (15g) were thoroughly mixed in a stainless steel bowl. Magnesium stearate (0.1g) was added as a lubricant.

The blended polymer was used to encase aspirin tablets each weighing 350mg and having a 10mm diameter and each containing 325mg of aspirin using a compression coating machine. The diameter of each compact coated tablet was 12mm and weighed approximately 600mg. The compact coated tablets were heated at 80°C - 90°C for 15 to 20 min to allow the polymer particles to fuse together to form a continuous film.

The film coated tablets were tested for enteric integrity using the USP procedure. The tablets remained intact in simulated gastric fluid (SGF) for more than two hours, and dissolved within 30min in the intestinal medium. No leaching out of aspirin was detected in the SGF.

### Example 2

The procedure of Example 1 was repeated using cellulose acetate phthalate (85g) as the polymer, glyceryl triacetate (15g) as the plasticizer and carbowax 6000 (0.1g) as lubricant. The results of enteric integrity testing were similar to those of Example 1.

### Example 3

The procedure of Example 1 was repeated using hydroxypropyl methylcellulose phthalate (85g) as polymer and diethyl phthalate (15 g) as plasticizer. The results of enteric integrity testing were acceptable.

Hydroxypropyl methylcellulose phthalate also gave good results with Triacetin, ethyl lactate, triethyl citrate and dibutyl sebacate.

### Example 4

The dissolution time in the intestinal medium increased with an increase in the thickness of the Aquateric polymer coat. When aspirin tablets each weighing 100mg and having a diameter of 6.4mm were coated with 200mg of the polymer blend of Example 1 to give a coating approximately 1 mm thick the tablets dissolved in the intestinal medium within 40min. Identical tablets were coated with 400mg of the polymer blend, giving a coating thickness of approximately 2mm and the dissolution time was increased from 40min to 90min.

### Example 5

Eudragit L100* (70g) or Eudragit S100** (70g) was mixed with propylene glycol (30g). This mixture was used to encase glucose tablets, each having a diameter of 6.4mm and containing glucose (100mg), amaranth dye (0.1mg) and magnesium stearate (1mg). The tablets were heated at 100°C for 5 minutes to effect fusion of the polymer particles. The film coat weight was 120mg and the thickness approximately 2mm. The coated tablets remained intact in SGF for more than 1 hour and dissolved within 2¹/₂ hour in simulated intestinal fluid.
* Eudragit L100 is a 1:1 copolymer of methacrylic acid and methyl methacrylate.
** Eudragit S100 is a 1:2 copolymer of methacrylic acid and methyl methacrylate.

### Example 6

The addition of a water insoluble polymer, cellulose acetate 398-10, to the enteric polymer cellulose acetate phthalate and a plasticizer, triacetin, prolonged the dissolution time of the enteric film coat and hence delayed the exposure of cores to the intestinal fluid. Increasing the proportion of cellulose acetate 398-10 in the blend increased the dissolution or erosion time in simulated intestinal fluid of the enteric coat on glucose cores having a 6.4mm diameter and containing 100mg glucose, 0.1mg amaranth dye and 1mg magnesium stearate. (Table 1).

**Table 1**

| Cellulose acetate phthalate (g) | Cellulose acetate 398-10 (g) | Triacetin (g) | Dissolution Time of film coat in simulated intestinal fluid (min) |
|---|---|---|---|
| 85 | 0 | 15 | 15 |
| 80 | 5 | 15 | 60 |
| 75 | 10 | 15 | 300 |
| 65 | 20 | 15 | 420 |

In each case the film coat weighed 250mg and was approximately 2mm in thickness. After encasing the cores with the polymer mix on a compression machine, the coated tablets were heated for 15 minutes at 90°C to effect fusion of the polymer particles.

All of the coated tablets met the resistant test in simulated gastric fluid for more than one hour.

### Example 7

A mixture of salbutamol sulphate (3.2g), calcium sulphate (16.66g) and amaranth (0.033g) was prepared and combined with a thoroughly mixed blend of cellulose acetate phthalate (64g) and Triacetin (16g). The mixture was used to compress 8mm diameter tablets each weighing 300mg and having a thickness of 4.5mm. The tablets were then coated as described in Example 2, which increased their weight to 420mg and their overall thickness by 1mm. The coated tablets were heated for 10 minutes at 100°C. The hardness of the tablets increased to 33Kp from 9Kp before heating.

The tablets remained resistant to simulated gastric fluid for more than 2 hours but dissolved slowly (4 hours) in simulated intestinal fluid.

### Example 8

Aspirin (2g) was combined with a thoroughly mixed blend of Aquateric (2g) and triacetin (0.5g). The mixture was used to compress 8.5mm diameter tablets, each weighing 500mg and having a thickness of 7.5mm. The tablets were coated as described in Example 1 which increased their weight to approximately 700mg and their overall thickness by approximately 1mm. The tablets were heated at 100°C for 10 minutes. The hardness of the tablets increased to 33Kp from 11Kp before heating.

The tablets remained resistant to simulated gastric fluid for more than 2 hours, but dissolved slowly (5 hours) in simulated intestinal fluid.

### Example 9

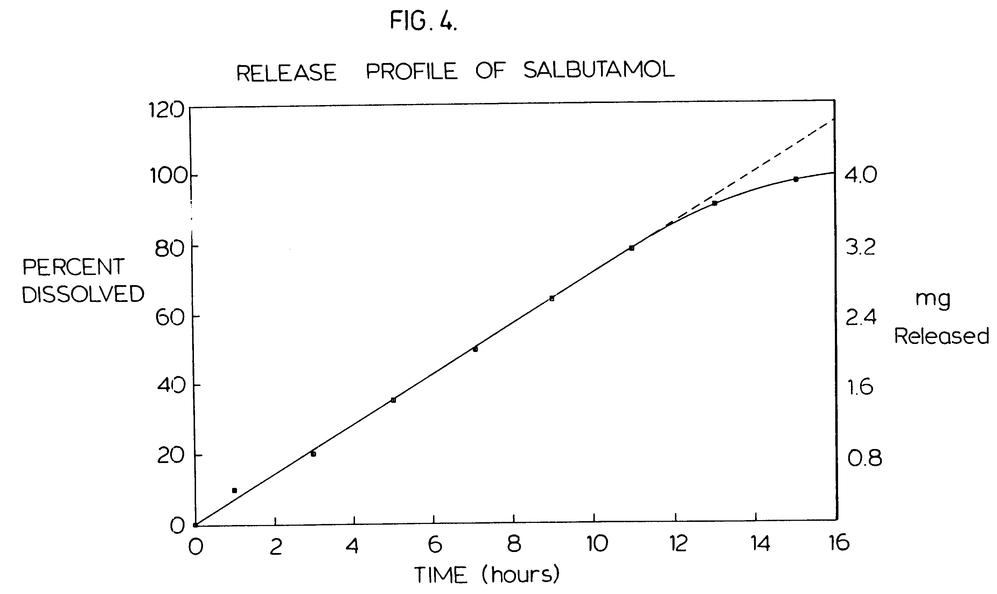

Tablet cores containing salbutamol (4mg) as salbutamol sulphate were manufactured by blending the drug with sodium chloride (75mg), Ac-di-sol* (1.8mg) and polyvinyl pyrrolidone (1.8mg) and granulating with a solution containing ethanol and water. The granules were dried, sized to 20 mesh and then blended with magnesium stearate (0.85mg). The blend was compressed on a single punch machine. The 6.4mm diameter cores produced were 1.5mm thick and weighed 85 mg. Cellulose acetate (70g) containing 39.8% acetyl groups, triacetin (30g) and polyoxyethylene sorbitan mono-oleate (3g) were thoroughly mixed in a stainless steel bowl. The mixture was allowed to stand at room temperature for a minimum of 24h before using it to encase the cores, described above, on a compression machine using an 8mm diameter punch as described in Figure 2. The polymer coated tablets were heated at 100°C for 10 min. The coated tablets were 2.26mm in thickness and weighed 165mg. The dissolution release profile of these tablets was obtained using the USP Apparatus 2. The graph shown in Figure 4 depicts a constant release profile of the drug over a 12 h period, after which the release rate begins to fall. More than 80% of the total drug was released within 12h at a rate of 0.28 mg/h. The remaining drug is released at a decreasing rate within the next 3 h. The correlation coefficient of the straight line relationship for the first 12 h was 0.9988. The membrane formed was porous in nature, the release of the drug in the absence of a delivery orifice confirmed this. Further confumation was obtained by evaluating the membrane in a diffusion cell.
* Ac-di-sol is a form of cross-linked carboxymethyl cellulose

## Claims

1. A method for the production of a solid pharmaceutical preparation characterised by mixing together finely divided particles or granules of a medicament, or granules containing a medicament and excipients, and appropriate amounts of a finely divided amorphous or substantially amorphous coating polymer and plasticizer which dissolves the polymer, compressing the mixture into tablets, and heating the tablets to a temperature at which fusion of the polymer particles occurs.

2. A method for the production of a solid pharmaceutical preparation characterised in that a solid core is coated with a film coat by mixing or granulating finely divided particles of a membrane-forming amorphous or substantially amorphous polymer with a plasticizer which dissolves the polymer, encasing said solid core with the polymer mix, and heating the polymer-encased material to a temperature at which the amorphous or substantially amorphous polymer forms a continuous film.

3. A method for the production of a solid pharmaceutical preparation as defined in claim 2 wherein, concomitant with the encasing process, the polymer coating is pierced at one or more points.

4. A method for the production of a solid pharmaceutical preparation as defined in claims 1 or 2 wherein the polymer is an enteric or macroporous film-forming polymer.

5. A method for the production of a solid pharmaceutical preparation as defined in claims 1 to 3 wherein the polymer is a semi-permeable or microporous film-forming polymer.

6. A method for the production of a solid pharmaceutical preparation according to claims 1 to 5 wherein the polymer encased/ compressed material is heated to a temperature in the range of 80 to 140°C.

7. A method for the production of a solid pharmaceutical preparation according to claims 1 to 6 wherein the encasing/compression is effected by means of a compression coating machine.

## Patentansprüche

1. Verfahren zur Herstellung eines festen pharmazeutischen Präparats, dadurch **gekennzeichnet,** daß man feinzerteilte Teilchen oder Körner eines Arzneimittels oder Körner, die ein Arzneimittel und Exzipientien enthalten, und geeignete Mengen eines feinzerteilten amorphen oder im wesentlichen amorphen Beschichtungspolymeren und eines Weichmachers, der das Polymere auflöst, vermischt, das Gemisch zu Tabletten verpreßt und die Tabletten auf eine Temperatur erhitzt, bei der eine Fusion der Polymerteilchen stattfindet.

2. Verfahren zur Herstellung eines festen pharmazeutischen Präparats, dadurch **gekennzeichnet,** daß man einen festen Kern mit einem Filmüberzug beschichtet, indem man feinzerteilte Teilchen eines membranbildenden amorphen oder im wesentlichen amorphen Polymeren mit einem Weichmacher, der das Polymere auflöst, vermischt oder granuliert, den festen Kern mit dem Polymergemisch umschließt und daß man das von dem Polymeren umschlossene Material auf eine Temperatur erhitzt, bei der das amorphe oder im wesentlichen amorphe Polymere einen kontinuierlichen Film bildet.

3. Verfahren zur Herstellung eines festen pharmazeutischen Präparats nach Anspruch 2, dadurch **gekennzeichnet,** daß man gleichzeitig mit dem Umschließungsprozeß den Polymerüherzug an ein oder mehreren Punkten perforiert.

4. Verfahren zur Herstellung eines festen pharmazeutischen Präparats nach Anspruch 1 oder 2, dadurch **gekennzeichnet,** daß das Polymere ein Polymeres ist, das einen darmlöslichen oder makroporösen Film bildet.

5. Verfahren zur Herstellung eines festen pharmazeutischen Präparats nach einem der Ansprüche 1 bis 3, dadurch **gekennzeichnet,** daß das Polymere ein semipermeables oder mikroporöses filmbildendes Polymeres ist.

6. Verfahren zur Herstellung eines festen pharmazeutischen Präparats nach einem der Ansprüche 1 bis 5, dadurch **gekennzeichnet,** daß man das Polymer-umschlossene/komprimierte Material auf eine Temperatur im Bereich von 80 bis 140°C erhitzt.

7. Verfahren zur Herstellung eines festen pharmazeutischen Präparats nach einem der Ansprüche 1 bis 6, dadurch **gekennzeichnet,** daß man das Umschließen/Komprimieren mittels einer Kompressions-Beschichtungsmaschine bewirkt.

## Revendications

1. Procédé de production d'une préparation pharmaceutique solide, caractérisé en ce qu'on mélange ensemble des particules ou granules finement divisées d'un médicament ou des granules contenant un médicament et des excipients et des quantités appropriées de polymère d'enrobage amorphe ou sensiblement amorphe finement divisé et de plastifiant **qui** dissout le polymère, on comprime le mélange pour former des comprimés et on chauffe les comprimés à une température à laquelle se produit la fusion des particules du polymère.

2. Procédé de production d'une préparation pharmaceutique solide, caractérisé en ce qu'on enrobe un noyau solide avec une pellicule d'enrobage en mélangeant ou en granulant des particules finement divisées d'un polymère amorphe ou sensiblement amorphe formant membrane avec un plastifiant qui dissout le polymère, on enrobe ledit noyau solide avec le mélange polymère et on chauffe la matière enrobée de polymère à une température à laquelle le polymère amorphe ou sensiblement amorphe forme une pellicule continue.

3. Procédé de production d'une préparation pharmaceutique solide selon la revendication 2, dans lequel, simultanément avec le procédé d'enrobage, on perce l'enrobage polymère en un ou plusieurs point(s).

4. Procédé de production d'une préparation pharmaceutique solide selon la revendication 1 ou 2, dans lequel le polymère est un polymère filmogène entérique ou macroporeux.

5. Procédé de production d'une préparation pharmaceutique solide selon les revendications 1 à 3, dans lequel le polymère est un polymère filmogène semi-perméable ou microporeux.

6. Procédé de production d'une préparation pharmaceutique solide selon les revendications 1 à 5, selon lequel on chauffe le matériau comprimé enrobé de polymère à une température comprise entre 80 et 140°C.

7. Procédé de production d'une préparation pharmaceutique solide selon les revendications 1 à 6, dans lequel on effectue l'enrobage et la compression à l'aide d'un appareil d'enrobage par compression.
